# EUROPEAN PATENT APPLICATION

(11) **EP 3 109 819 A1**
(43) Date of publication of application: **28.12.2016**
(21) Application number: 15752763.1
(22) Date of filing: 18.02.2015
(51) Int. Cl.: G06Q 50/22

(54) **SLEEP IMPROVEMENT SUPPORT DEVICE, SLEEP IMPROVEMENT SUPPORT METHOD, SLEEP IMPROVEMENT SUPPORT PROGRAM, AND SLEEP IMPROVEMENT SUPPORT PROGRAM RECORDING MEDIUM**

(30) Priority: 19.02.2014 JP 2014029709
(71) Applicant: NEC Solution Innovators, Ltd., Tokyo 136-8627 (JP)
(72) Inventor: YAMAMOTO, Kosuke, Tokyo 136-8627 (JP); OKAJIMA, Isa, Kodaira-shi Tokyo 187-0013 (JP)
(74) Representative: Betten & Resch
(86) International application number: PCT/JP2015/000771
(87) International publication number: WO 2015/125477

(57) **Abstract**

The challenge addressed by the invention is, in a case in which a consulter harbors a mistaken perception regarding the quality of his or her own sleep, to bring this to the attention of the consulter in a simple manner. In order to resolve this challenge, a sleep improvement support device is provided with: a sleep-related data storage unit (104) which stores two or more days' worth of sleep-related data including date information, sleep evaluation data for measuring the quality of the day's sleep, and quality evaluation data influenced by the quality of the day's sleep; a main complaint information input unit (101) which accepts as input main complaint information indicating the main complaint of the consulter; a contradiction information extracting unit (102) which extracts, from the stored sleep-related data, contradiction information indicating differences by day in the relationship between the data for a main complaint item, which is an item of sleep evaluation data corresponding to the main complaint of the consulter, and data for a main complaint evaluation item, which is an item of quality evaluation data corresponding to the main complaint of the consulter; and an output unit (103) which outputs the extracted contradiction information and/or a message based on the extracted contradiction information.

## Description

### Technical Field

The present invention relates to a sleep improvement support device, a sleep improvement support method, a sleep improvement support program, and a sleep improvement support program storage medium, which assist, with sleep improvements, people having insomnia or sleep-related problems.

### Background Art

A cognitive-behavioral therapy is known as a methodology for correcting a tendency to think things negative, into a balanced mind set, by focusing on the facts and behaviors. In the field of insomnia therapy, too, it has been reported that a therapeutic effect with a high remission rate is obtained by introducing the cognitive-behavioral therapy. The cognitive-behavioral therapy for insomnia is called CBT-I (cognitive-behavioral therapy for insomnia), and there also exist Web services that are based on the CBT-I concept and CBT-I tools such as a self-care application.

In CBT-I, guidance related to sleep hygiene, guidance related to sleep schedules and guidance related to relaxation are performed.

In the guidance related to sleep hygiene, practices and environments that would cause awakening are learned, and if there is such a practice or an environment, lifestyle improvement measures are taken to improve them, for example. In addition, in the guidance related to sleep schedules, importance of making it a practice to sleep on the bed or on the bedding is learned, and lifestyle improvement measures to practice such a practice. Furthermore, in the guidance related to relaxation, a mindset or a behavior so as to prepare for sleep without any unneeded power is learned, and lifestyle improvement measures incorporating a relaxation method of their own are taken.

Furthermore, in addition to the above-described guidance, there are cases where mental education is provided such as, analyzing the situation of the consulter and correcting knowledges regarding the factors relevant to the consulter's sleep is conveyed to the consulter, giving an advice or disclosing data so as to correct a habit in the consulter's cognition or behavior that could continue or worsen the insomnia, if any, and the like.

In CBT-I, the consulter is given, as a daily assignment, among the lifestyle improvement measures as described above, those that are not achieved by the consulter, and through exercising such assignments, the consulter's practices which have adverse effects on the consulter's sleep are removed. The consulter may temporarily have hard time coping with such assignments on a daily basis, but if the consulter can learn a proper practice, the sustainable effect can be expected.

As a technology making use of such a concept of CBT-I, Patent Literature 1, for example, describes a system that obtains a correlation between the collected objective indices related to sleep and the subjective indices, and makes a proposal to improve sleep on the basis of the result thereof.

In addition, Patent Literature 2 describes a system that provides a warning, a guide, an advice, or a message to encourage users, and proposes update of the behavior, so as to help the user comply with any behavioral program selected for the user.

### Citation List

### Patent Literature

[PTL 1] International Publication WO 2008/096307
[PTL 2] Japanese Patent No. 5307084

### Summary of Invention

### Technical Problem

CBT-I is known to be highly effective on condition that the consulter is dedicated to continue to achieve the daily assignments given to the consulter. However, CBT-I has such a problem that, when the consulter has a wrong cognition about the quality of his or her sleep, no effect is obtained even if he or she achieves the assignments, or the symptom worsens.

Among the symptoms alleged by a patient to a doctor, the main one is called "chief complaint". In the field of cognitive-behavioral therapy, the consulter's concern or worries are also called "chief complaint", not limited to symptoms. There are roughly four main complaints related to insomnia as follows:

### Difficulty falling asleep,

### Awaking during sleep

### Early morning awaking, and

### Trouble in sound sleep.

Examples of the chief complaint belonging to "difficulty falling asleep" include "not easily falling asleep". Examples of the chief complaint belonging to "awaking during sleep" include "awaking often during sleep". Examples of the chief complaint belonging to "early morning awaking" include "awaking early in the morning". Examples of the chief complaint belonging to "trouble in sound sleep" include "not feeling like having slept."

The chief complaint is based on the subjective judgment of the consulter. When the consulter takes the chief complaint excessively serious, there may occur such cases where, even if the consulter achieves the assignment, the effect is not felt, which hinders the achievement of the assignment, or such cases where the worries worsen to cause the consulter to take the mindset or the behavior that would tend to cause insomnia. In order to avoid such problems, it is effective to remind the consulter that the state alleged by the consulter as the chief complaint is not in fact affecting the daily lives so much as the consulter thinks.

Note that the method described in Patent Literature 1 is merely to make a proposal effective for the subjective indices, by finding an objective index correlated with the subjective indices, and is not intended to assist the consulter by focusing on no correlation between the chief complaint and the state of the next day.

In Patent Literature 2, through analyzing the subjective information and the objective information, it is possible to show that a sufficient amount of sleep is obtained using the objective information, for example, even when a user report the incidence of difficulty staying asleep or unrefreshing sleep, and the activity level during the day is scored low. The objective information is, for example, sensor information sensing the activities at night and a cognition score of a user. The described is that this can resolve anxiety of a user.

However, in order to implement the method described in Patent Literature 2, the sensor unit data or the objective test data are necessary, and, accordingly, there are problems that a large-scale system is required to collect them, and they are troublesome for a user since a test have to be taken.

In view of this, the present invention has an objective of providing a sleep improvement support device, a sleep improvement support method, a sleep improvement support program, and a sleep improvement support program storage medium which are capable of, when a consulter has a wrong cognition about the quality of the consulter's own sleep, easily making the consulter notice that.

### Solution to Problem

A sleep improvement support device according to the present invention includes: sleep-related data storage means for storing, for two or more days, with date information, sleep-related data including sleep evaluation data for measuring a quality of sleep of a day and quality evaluation data that is influenced by the quality of sleep of the day, the sleep evaluation data being data of one or more items each of which indicates a state, a behavior, or a sense of a consulter, the quality evaluation data being data of one or more items each of which indicates a state, a behavior, or a sense of the consulter after rising; chief complaint information input means for receiving, as input, chief complaint information indicating chief complaint of the consulter; contradiction information extracting means for extracting contradiction information indicating variation, between days, in relationship between chief complaint item data and chief complaint evaluation item data, from the sleep-related data stored in the sleep-related data storage means, the chief complaint item data being an item of sleep evaluation data relating to the chief complaint of the consulter, the chief complaint evaluation item data being an item of quality evaluation data relating to the chief complaint of the consulter; and output means for outputting the contradiction information extracted by the contradiction information extracting means and/or a message based on the contradiction information.

A sleep improvement support method according to the present invention includes: in a state in which sleep-related data storage means stores, for two or more days, with date information, sleep-related data including sleep evaluation data for measuring a quality of sleep of a day and quality evaluation data that is influenced by the quality of sleep of the day, the sleep evaluation data being data of one or more items each of which indicates a state, a behavior, or a sense of a consulter, the quality evaluation data being data of one or more items each of which indicates a state, a behavior, or a sense of the consulter after rising, when chief complaint information indicating chief complaint of the consulter is received as input, extracting, by an information processing device, contradiction information indicating variation, between days, in relationship between chief complaint item data and chief complaint evaluation item data, from the sleep-related data stored in the sleep-related data storage means, the chief complaint item data being an item of sleep evaluation data relating to the chief complaint of the consulter, the chief complaint evaluation item data being an item of quality evaluation data relating to the chief complaint of the consulter; and outputting, by the information processing device, the contradiction information extracted by the contradiction information extracting means and/or a message based on the contradiction information.

A program storage medium the present invention stores a sleep improvement support program causing a computer capable of accessing sleep-related data storage means storing, for two or more days, with date information, sleep-related data including sleep evaluation data for measuring a quality of sleep of a day and quality evaluation data that is influenced by the quality of sleep of the day, the sleep evaluation data being data of one or more items each of which indicates a state, a behavior, or a sense of a consulter, the quality evaluation data being data of one or more items each of which indicates a state, a behavior, or a sense of the consulter after rising, to execute: chief complaint information input processing of receiving, as input, chief complaint information indicating chief complaint of the consulter; contradiction information extracting processing of extracting contradiction information indicating variation, between days, in relationship between chief complaint item data and chief complaint evaluation item data, from the sleep-related data stored in the sleep-related data storage means, the chief complaint item data being an item of sleep evaluation data relating to the chief complaint of the consulter, the chief complaint evaluation item data being an item of quality evaluation data relating to the chief complaint of the consulter; and output processing of outputting the contradiction information extracted by the contradiction information extracting means and/or a message based on the contradiction information.

### Advantageous Effects of Invention

According to the present invention, it is possible to easily make, when a consulter has a wrong cognition about the quality of the consulter's own sleep, the consulter notice that.

### Brief Description of Drawings

[Fig. 1] Fig. 1 is a block diagram illustrating an exemplary configuration of a sleep improvement support device according to a first exemplary embodiment.
[Fig. 2] Fig. 2 is an explanatory diagram illustrating an example of items described in a sleep diary.
[Fig. 3] Fig. 3 is an explanatory diagram illustrating an example of sleep-related data stored in a sleep-related data storage means 104.
[Fig. 4] Fig. 4 is an explanatory diagram illustrating an example of chief complaint correspondence information.
[Fig. 5] Fig. 5 is a flowchart illustrating an exemplary operation of the sleep improvement support device according to the first exemplary embodiment.
[Fig. 6] Fig. 6 is an explanatory diagram illustrating an example of an extracting method of contradiction information.
[Fig. 7] Fig. 7 is an explanatory diagram illustrating an example of a cross-tabulation table.
[Fig. 8] Fig. 8 is an explanatory diagram illustrating an example of affirmation-negation definition data.
[Fig. 9] Fig. 9 is an explanatory diagram illustrating an example of a non-correlation notification message 12.
[Fig. 10] Fig. 10 is an explanatory diagram illustrating an output example of an output means 103.
[Fig. 11] Fig. 11 is an explanatory diagram illustrating an example of consistency definition data.
[Fig. 12] Fig. 12 is an explanatory diagram illustrating an example of sleep-related data.
[Fig. 13] Fig. 13 is an explanatory diagram illustrating an extracting method of contradiction information.
[Fig. 14] Fig. 14 is an explanatory diagram illustrating an output example of the output means 103.
[Fig. 15] Fig. 15 is a block diagram illustrating an exemplary configuration of a sleep improvement support device according to a second exemplary embodiment.
[Fig. 16] Fig. 16 is a flowchart illustrating an exemplary operation of the sleep improvement support device according to the second exemplary embodiment.
[Fig. 17] Fig. 17 is an explanatory diagram illustrating an example of priority level assigned by a contradiction information ranking means 201.
[Fig. 18] Fig. 18 is an explanatory diagram illustrating an example of consistency definition data according to the second exemplary embodiment.
[Fig. 19] Fig. 19 is an explanatory diagram illustrating a calculation expression of the priority level.
[Fig. 20] Fig. 20 is an explanatory diagram illustrating an output example of the output means 103.
[Fig. 21] Fig. 21 is a block diagram illustrating another exemplary configuration of the sleep improvement support device according to the second exemplary embodiment.

### Description of Embodiments

### First Exemplary Embodiment.

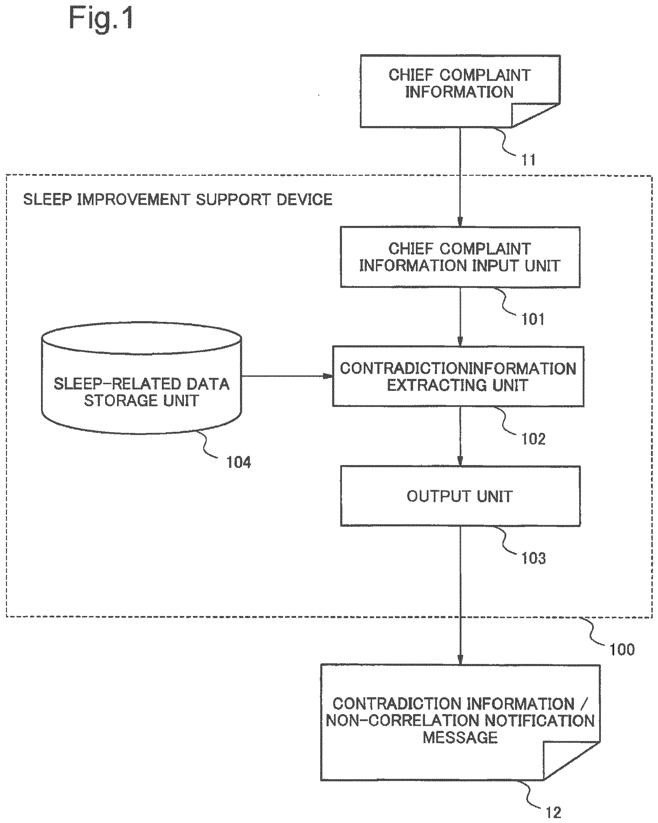

Fig. 1 is a block diagram illustrating an exemplary configuration of a sleep improvement support device according to a first exemplary embodiment of the present invention. A sleep improvement support device 100 illustrated in Fig. 1 includes a chief complaint information input unit 101, a contradiction information extracting unit 102, an output unit 103, and a sleep-related data storage unit 104.

The sleep-related data storage unit 104 stores, for two or more days, together with information on date, sleep-related data including: sleep evaluation data that is data of one or more items indicating a state or a sense of a consulter for measuring a quality of sleep on a day; and quality evaluation data that is data of one or more items indicating a state or a sense of the consulter after waking up, which is influenced by the quality of sleep on the day.

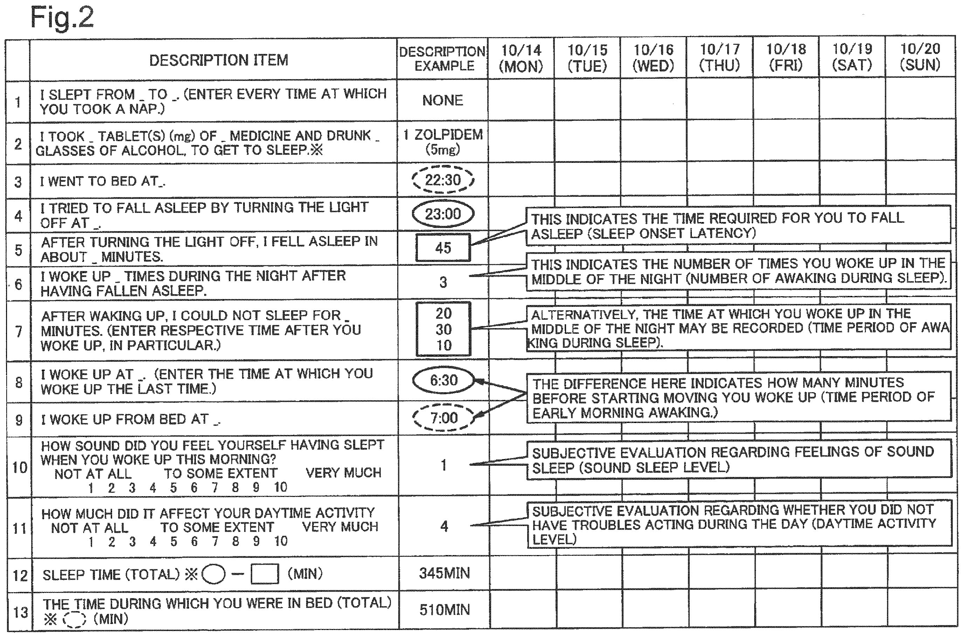

The sleep-related data, for example, may be a group of data related to the items as described in a sleep diary illustrated in Fig. 2. Fig. 2 is an explanatory diagram illustrating an example of items described in a sleep diary. Generally speaking, the items described in a sleep diary include quality evaluation data. The quality evaluation data is data for evaluating sleep evaluation data that is basic data for measuring the quality of sleep of the consulter on a day and the quality of sleep after waking up. It also includes, for example, data of the following items:
(1) Time when taking a nap; existence of an act of taking medicine, a type of the medicine, and an amount of the medicine; existence of an act of drinking alcohol and an amount of the alcohol; and time when going to bed;
(2) Time when trying to sleep, and a time period taken to fall asleep (sleep onset latency);
(3) The number of times of awaking during sleep (the number of awaking during sleep) and a time period of awaking during sleep (time of awaking during sleep);
(4) Time period from awaking till starting moving to (time period of early morning awaking);
(5) Subjective evaluation regarding feelings of sound sleep (sound sleep level); and
(6) Subjective evaluation regarding how much daytime (after-awaking) activities are carried out (daytime activity level).

In the present exemplary embodiment, the sleep-related data includes, as sleep evaluation data, data of one or more items indicating a state, behavior or a sense of a consulter regarding sleep on a day. In addition, the sleep-related data includes, as quality evaluation data, data of one or more items indicating a state, behavior or a sense of the consulter after awaking. The sleep evaluation data may be basic data regarding sleep of the consulter. The quality evaluation data may be data indicating a state, behavior or a sense which is of the consulter and is influenced by the quality of sleep, and may preferably include, in particular, data indicating a state, behavior or a sense which is of the consulter and is directly influenced when getting symptoms that tends to be alleged as a chief complaint.

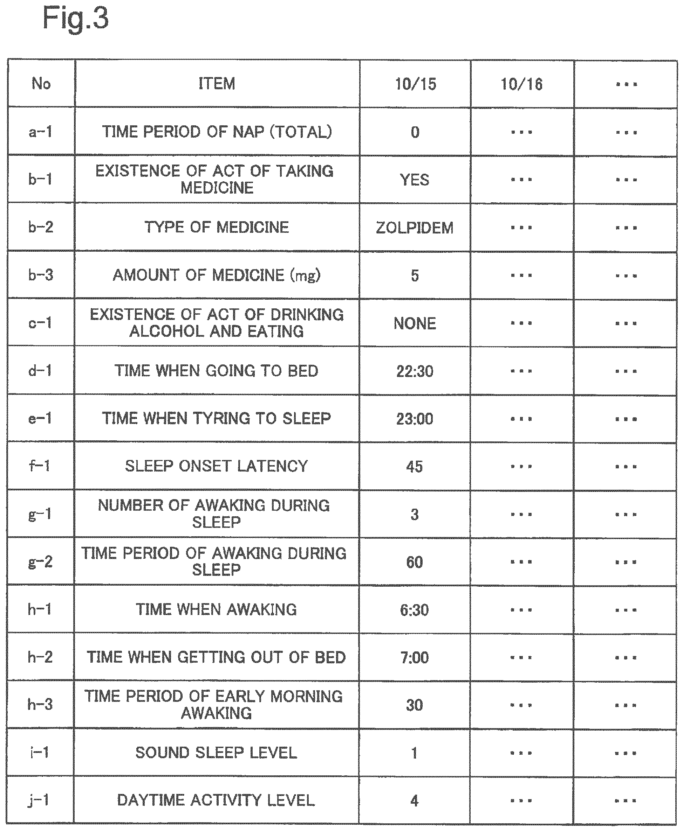

Fig. 3 is an explanatory diagram illustrating an example of sleep-related data stored in the sleep-related data storage unit 104. Fig. 3 represents an example of the sleep-related data in which an item number by which an item included in the sleep-related data is identified and an explanation of the item are associated with details of the item separately for each different date. In this example, data of "daytime activity level" that is the item having the item ID = "j-1" (hereafter referred to as "daytime activity level data") corresponds to the quality evaluation data, and the data of the other items corresponds to the sleep evaluation data.

The chief complaint information input unit 101 receives, as input, chief complaint information indicating a chief complaint of the consulter. Hereafter, the chief complaint information input by the chief complaint information input unit 101 is referred to as chief complaint information 11.

The contradiction information extracting unit 102 extracts contradiction information indicating daily variation in relationship between data of chief complaint items that are items of the sleep evaluation data corresponding to a chief complaint of a consulter (hereinafter referred to as "chief complaint item data") and data of chief complaint evaluation items that are items of the quality evaluation data corresponding to the chief complaint of the consulter (hereinafter referred to as "chief complaint evaluation item data"), from among the sleep-related data stored in the sleep-related data storage unit 104. A type of correlation (positive correlation or negative correlation) when assuming that there is a correlation may be considered as an example of the relationship between the chief complaint item data and certain chief complaint evaluation item data.

For example, for each of chief complaint types which are predetermined, chief complaint correspondence information in which a chief complaint item and a chief complaint evaluation item are associated is stored in a predetermined storage apparatus. Then, the chief complaint information input unit 101 receives the chief complaint information including a chief complaint type of a chief complaint of a consulter. In such a case, the contradiction information extracting unit 102 may extract the contradiction information indicating the daily variation of the relationship between the chief complaint item data and the chief complaint evaluation item data, based on the input chief complaint information 11 and the chief complaint correspondence information. The chief complaint types indicate predetermined types into which complaints that are possible as the chief complaints are classified in advance, and are the above-mentioned "difficulty falling asleep" "awaking during sleep" "early morning awaking" and " trouble in sound sleep" and the like. The chief complaint types are not limited to those. Although not illustrated in Fig. 1, the sleep improvement support device 100 according to the present exemplary embodiment may include a chief complaint correspondence information storage unit storing therein such chief complaint correspondence information.

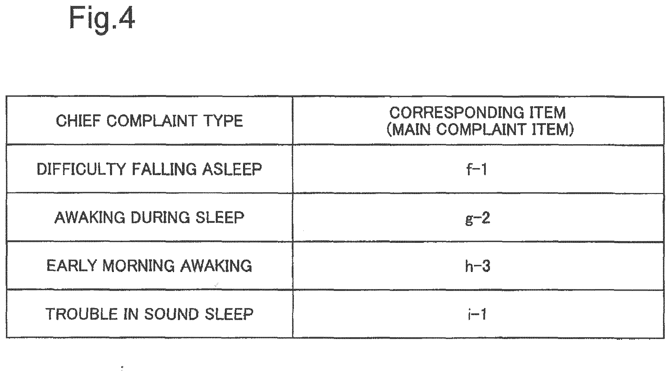

Fig. 4 is an explanatory diagram illustrating an example of chief complaint correspondence information. Fig. 4 illustrates an example of the chief complaint correspondence information in which a chief complaint type is associated with an item ID of a relevant item of the chief-complaint-related data as information indicating the chief complaint item. The chief complaint correspondence information may be information in which a chief complaint evaluation item that a relevant item in the quality evaluation data is associated, in addition to the chief complaint item. One chief complaint evaluation item may be predetermined regardless of the chief complaint type, or the user may select the chief complaint evaluation item. When a user selects the chief complaint evaluation item, the chief complaint information input unit 101 may receive the chief complaint information 11 including the selected chief complaint evaluation item.

The output unit 103 outputs the contradiction information extracted by the contradiction information extracting unit 102. Or, the output unit 103 outputs a message based on the contradiction information extracted by the contradiction information extracting unit 102. Hereinafter, the message based on the contradiction information output by the output unit 103 may be referred to as non-correlation notification message 12. The output unit 103 may output the contradiction information as it is, or may output, as non-correlation notification message 12, a message indicating that there is no correlation between the chief complaint item data and the chief complaint evaluation item data, together with information indicating how much the variation of the relationship between the chief complaint item data and the chief complaint evaluation item data is, for example.

According to the present exemplary embodiment, the sleep-related data storage unit 104 is implemented with a storage unit such as a memory or a database system. In addition, the chief complaint information input unit 101 is implemented with an information input apparatus such as a mouse and a keyboard, or a touch panel, and an information processing apparatus such as a CPU (central processing unit) operating according to a program. The contradiction information extracting unit 102 is implemented with an information processing apparatus such as a CPU operating according to a program. In addition, the output unit 103 is implemented with an information output apparatus such as a display apparatus or a printer, and an information processing apparatus such as a CPU operating according to a program. The information input apparatus and the information output apparatus include a network interface such as a network card that receives information from a connected communication network and transmits information to the connected communication network, and a control apparatus thereof.

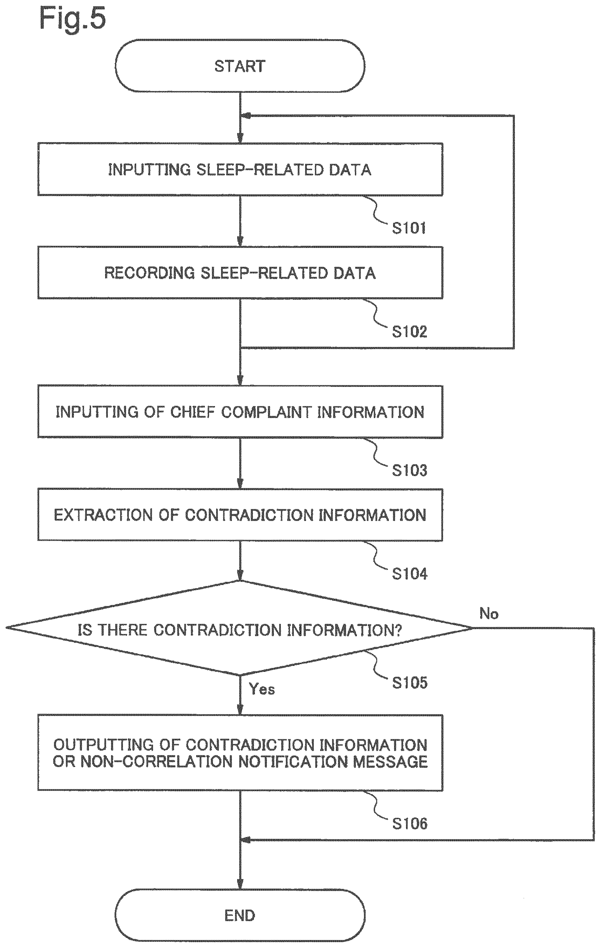

Next, the operation according to the present exemplary embodiment is explained. Fig. 5 is a flowchart illustrating an exemplary operation according to the present exemplary embodiment. As illustrated in Fig. 5, when sleep-related data is input to the sleep improvement support device 100 according to the present exemplary embodiment, the sleep-related data storage unit 104 successively stores the input sleep-related data together with date information (Steps S101, S102). Although not illustrated in Fig. 1, the sleep improvement support device 100 may include a sleep-related data input unit prompting a user to input sleep-related data. The sleep-related data input unit may, for example, provide an interface (input screen, and the like) to a user for inputting the content of sleep-related data for one day, and register, to the sleep-related data storage unit 104, content of each item input through the interface, as the sleep-related data of the designated day.

When the sleep-related data storage unit 104 is in a state of storing therein sleep-related data of two or more days, and chief complaint information 11 is input through the chief complaint information input unit 101 (Step S103), the contradiction information extracting unit 102 extracts contradiction information from the sleep-related data stored in the sleep-related data storage unit 104 (Step S104).

The chief complaint information input unit 101, for which an interface for a user to input a concern is prepared in advance, may input the chief complaint information 11, for example, by prompting the user to select the relevant one on the interface from among alternatives of the chief complaint types included in the chief complaint, which are set as options. The chief complaint information input unit 101 may have a function of analyzing the characters freely input by a user, and automatically determining which of the chief complaint types is relevant. In such a case, data for recognition such as keywords and expression samples each relating to the chief complaint types, for example, "not easily falling asleep", "awaking during sleep", "awaking early in the morning", "not feeling like having slept" and the like, may be registered in advance in the chief complaint correspondence information.

When the contradiction information extracting unit 102 extracts contradiction information, the output unit 103 presents (outputs), to a user, the extracted contradiction information and/or the non-correlation notification message 12 based on the extracted contradiction information (Yes in Step S105, S106). When no contradiction information is extracted, the process may just end (No in Step S105).

Next, an extracting method of contradiction information by the contradiction information extracting unit 102 is explained with reference to a specific example.

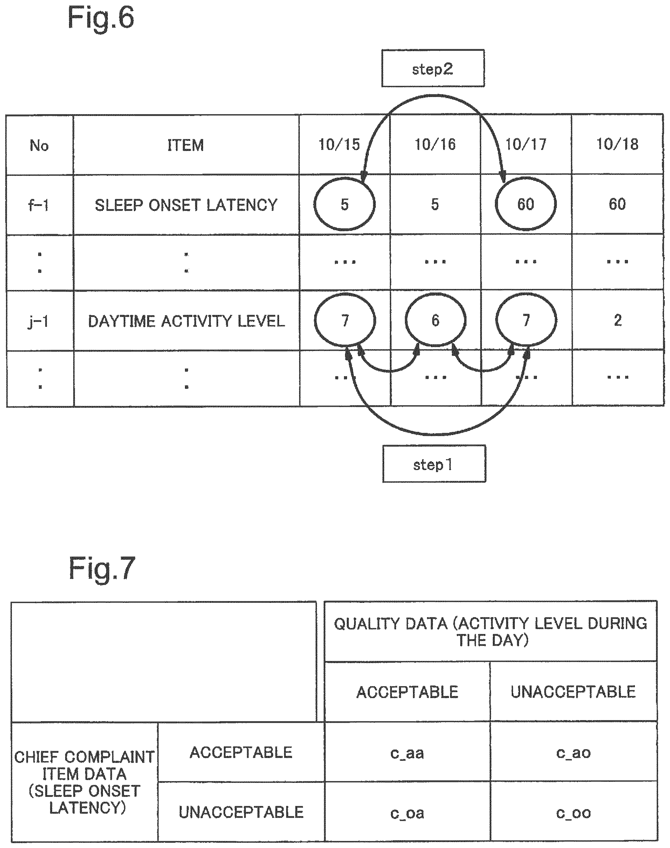

Fig. 6 is an explanatory diagram illustrating an example of an extracting method of contradiction information. In the following, explanation is given using, as an example, a case in which the chief complaint information 11 including the chief complaint type indicating "difficulty falling asleep" is input. In addition, in the following, it is assumed that, as illustrated in Fig. 4, "sleep onset latency" that is the item of the item ID = "f-1" is registered in the chief complaint correspondence information, as a chief complaint item associated with "difficulty falling asleep". It is also assumed that "daytime activity level" that is the item of the item ID="j-1" is set, in advance, to be used as a chief complaint evaluation item associated with "difficulty falling asleep".

As illustrated in Fig. 6, the contradiction information extracting unit 102 first identifies a combination of dates (hereinafter referred to as "date pair") on which the similarity level of the chief complaint evaluation item data between the dates is equal to or more than a predetermined threshold value, from the sleep-related data stored in the sleep-related data storage unit 104 (Step 1). In Step 1, the date pair of "10/15" and "10/16", the date pair of "10/15" and "10/17", and the date pair of "10/16" and "10/17", which are three date pairs in total, are identified by assuming that the date pair has a similarity level equal to or more than the predetermined threshold value when the difference between the daytime activity levels indicated by the daytime activity level data of the dates in the date pair is within ±1.

Next, from among the identified date pairs, the contradiction information extracting unit 102 identifies the date pair for which difference in the chief complaint item data is equal to or more than a predetermined threshold value (Step 2). In Step 2, the date pair of "10/15" and "10/17" is selected, as the date pair having the largest difference of the sleep onset latency indicated by sleep onset latency data of the dates included in the date pair, from among the date pairs having the difference of the sleep onset latency is equal to or more than 30 minutes, for example. Lastly, the chief complaint item data in the date pair identified in Step 2 (the sleep onset latency data in this example) and the chief complaint evaluation item data (daytime activity level in this example) are extracted as the contradiction information (Step 3).

When contradiction information is extracted, the output unit 103 may output, together with the extracted contradiction information, a message indicating that the chief complaint ("difficulty falling asleep" in this example) is not a very serious problem.

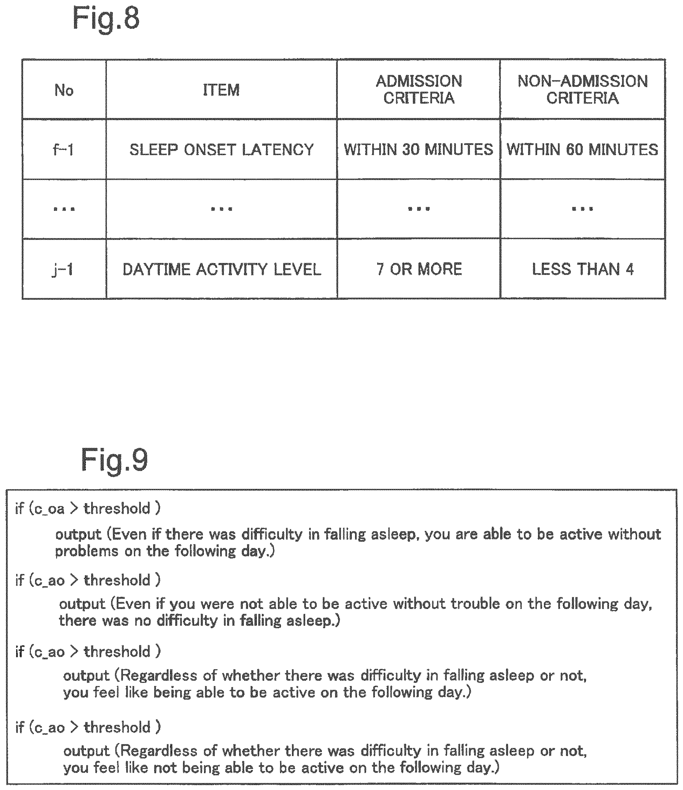

Next, with reference to Fig. 7 through Fig. 9, another extracting method of the contradiction information by the contradiction information extracting unit 102 is explained. Fig. 7 is an explanatory diagram illustrating an example of a cross-tabulation table used for extraction of the contradiction information. Fig. 8 is an explanatory diagram illustrating an example of affirmation-negation definition data used for extraction of the contradiction information. Fig. 9 is an explanatory diagram illustrating an output example of a non-correlation notification message 12 based on the extracted contradiction information.

On the basis of the affirmation-negation definition data as illustrated in Fig. 8 for example, the contradiction information extracting unit 102 performs cross-tabulation of the number of days of the chief complaint item data as each of true and false (admission/non-admission) and the number of days of the chief complaint evaluation item data as each of true and false (admission/non-admission), for the sleep-related data of dates of latest days of a predetermined number (e.g., seven days), among the sleep-related data stored in the sleep-related data storage unit 104. When, as a result of the cross-tabulation, the number of days on which the affirmation-negation does not match between the chief complaint item data and the chief complaint evaluation item data is equal to or more than a predetermined threshold value, the contradiction information extracting unit 102 may extract the result of that cross-tabulation as the contradiction information. The result of such cross-tabulation helps examine the level of how the relationship between the chief complaint item data and the chief complaint evaluation item data varies in different days.

The affirmation-negation definition data is data defining the affirmation-negation determining method of the items included in the sleep-related data. More specifically, the affirmation-negation definition data may be information defining the affirmation-negation determining method, regarding at least the chief complaint item and the chief complaint evaluation item included in the sleep-related data. Fig. 8 represents an example of affirmation-negation definition data including admission criteria and non-admission criteria, as an example of information representing the affirmation-negation determining method. Although not illustrated in Fig. 1, the sleep improvement support device 100 according to the present exemplary embodiment may include a affirmation-negation data storage unit storing therein such definition data.

On the basis of the affirmation-negation definition data illustrated in Fig. 8, the contradiction information extracting unit 102 counts the number of days on which the chief complaint item data satisfies admission criterion and the chief complaint evaluation item data satisfies admission criterion, in the sleep-related data of the latest seven days, and records the number of days in the field "c_aa" of the cross-tabulation table illustrated in Fig. 7. Similarly, the contradiction information extracting unit 102 counts the number of days on which the chief complaint item data satisfies the admission criterion and the chief complaint evaluation item data satisfies non-admission criterion, in the sleep-related data of the latest seven days, and records the number of days in the field "c_ao" of the cross-tabulation table illustrated in Fig. 7. Similarly, the contradiction information extracting unit 102 counts the number of days on which the chief complaint item data satisfies non-admission criterion and the chief complaint evaluation item data satisfies the admission criterion, in the sleep-related data of the last seven days, and records the number of days in the field "c_oa" of the cross-tabulation table illustrated in Fig. 7. Similarly, the contradiction information extracting unit 102 counts the number of days on which the chief complaint item data satisfies the non-admission criterion and the chief complaint evaluation item data satisfies the non-admission criterion, in the sleep-related data of the last seven days, and records the number of days in the field "c_oo" of the cross-tabulation table illustrated in Fig. 7. When, as a result of the measurement, the number of days in the field "c_oa" is equal to or more than a predetermined threshold or the number of days in the field "c_ao" is equal to or more than the predetermined threshold value, the contradiction information extracting unit 102 may extract, as contradiction information, the cross-tabulation table recording measurement results.

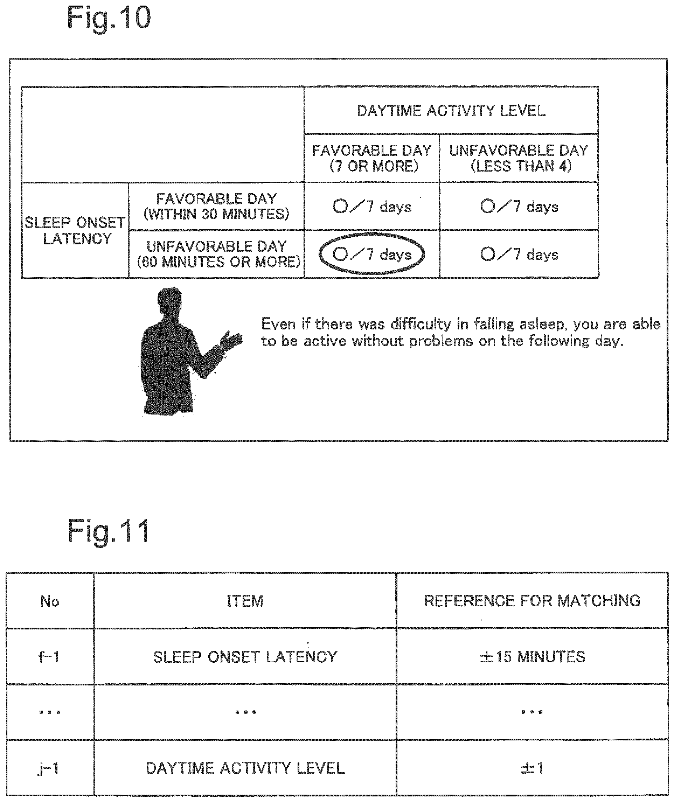

When the contradiction information is extracted, the output unit 103 may output a non-correlation notification message 12 as illustrated in Fig. 9, together with the extracted contradiction information, on the basis of the extracted contradiction information. For example, assume a case in which the number of days in the column "c_oa" is equal to or more than the predetermined threshold value, and there is a sleep state, which is of "Even if there was difficulty in falling asleep, you are able to be active without problems on the following day" or the like, relevant to the chief complaint. In such cases, the output unit 103 may output a non-correlation notification message 12 notifying that there are days on which the chief complaint evaluation item data that is naturally influenced indicates a good value. In this case, as illustrated in Fig. 10, it is preferable to display, in emphasis, the field "c_oa" in the cross-tabulation table. Accordingly, variation in relationship (how it varies) between the chief complaint item data and the chief complaint evaluation item data and the level of difference (how much it varies) can also be informed. The output unit 103 may only output the contradiction information (cross-tabulation table) in which a relevant part is displayed in emphasis, without outputting any message. If the user is a doctor and the like, when the relevant part is represented, the user can give an appropriate advice to the patient on the basis of it. Fig. 10 is an explanatory diagram illustrating an output example of an output unit 103 in this example.

Assume a case of a day on which the number of days in the field "c_ao" is equal to or more than the predetermined threshold value, and the chief complaint evaluation item data is bad, which is of "Even if you were not able to be active without trouble on the following day, there was no difficulty in falling asleep" or the like. Even if in such a case, the output unit 103 may output a non-correlation notification message 12 notifying that there is a day on which a sleep state relating to the chief complaint is good. In this case, it is preferable to display, in emphasis, the field "c_ao" in the cross-tabulation table. Also assume a case in which the number of days in the field "c_oa" is equal to or more than the predetermined threshold value and the number of days in the field "c_aa" is equal to or more than the predetermined threshold value, and there is a sleep state, which are of "Regardless of whether there was difficulty in falling asleep or not, you feel like being able to be active on the following day" or the like, relevant to the chief complaint. In such a case, the output unit 103 may output a non-correlation notification message 12 notifying that there is a day on which the chief complaint evaluation item data that is naturally influenced indicates a good value, and there is also another day on which the chief complaint evaluation item data indicates a bad value. In this case, it is preferable to display, in emphasis, the field "c_oa" and the field "c_aa" in the cross-tabulation table. Also assume a case in which the number of days in the field "c_ao" is equal to or more than the predetermined threshold value, the number of days in the field "c_oo" is equal to or more than the predetermined threshold value. In such cases, the output unit 103 may output a non-correlation notification message 12, "Regardless of whether there was difficulty in falling asleep or not, you feel like not being able to be active on the following day", notifying that there is a day on which a sleep state relating to the chief complaint is bad and there is a day on which a sleep state relating to the chief complaint is good, among the days on which the chief complaint evaluation item data is bad. In this case, it is prefereable to display, in emphasis, the field "c_ao" and the field "c_oo" in the cross-tabulation table.

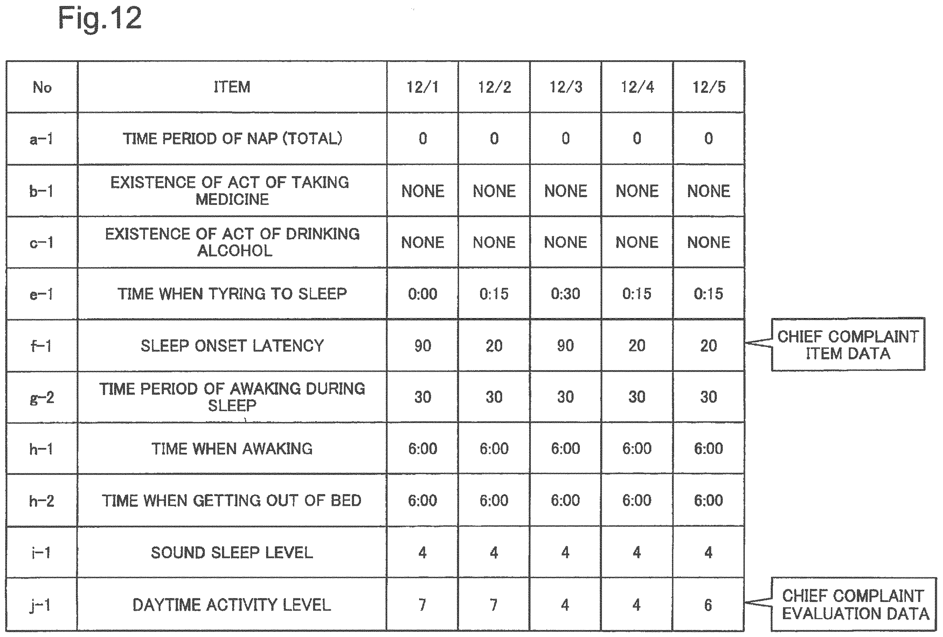

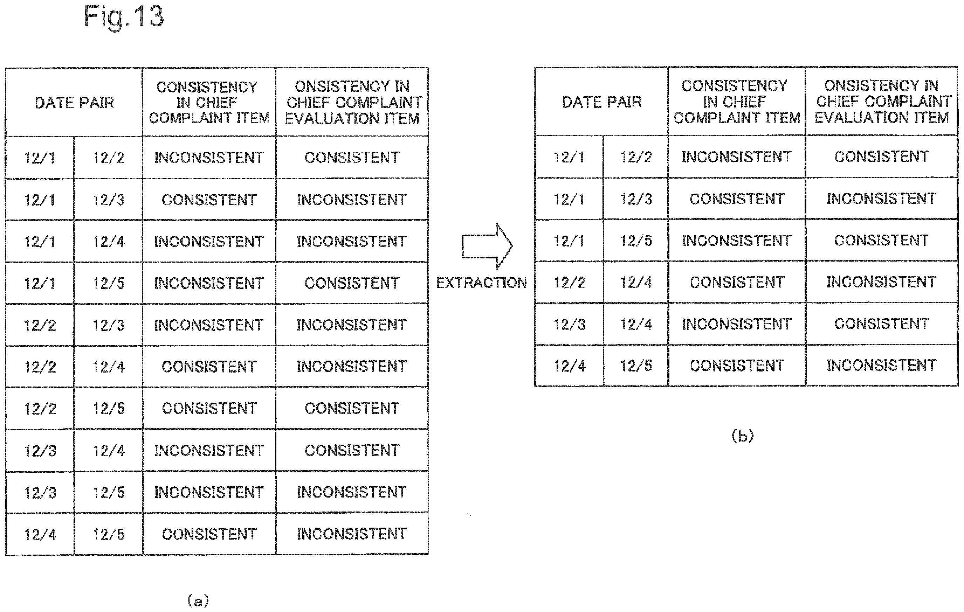

With reference to Fig. 11 through Fig. 13, another extracting method of the contradiction information by the contradiction information extracting unit 102 is explained. Fig. 11 is an explanatory diagram illustrating an example of consistency definition data used for extraction of contradiction information. Fig. 12 is an explanatory diagram illustrating an example of sleep-related data from which the contradiction information is extracted. Fig. 13 is an explanatory diagram illustrating an extracting method of contradiction information.

The contradiction information extracting unit 102 may extract contradiction information indicating that the relationship between the chief complaint item data relating to the chief complaint of the consulter and predetermined chief complaint evaluation item data varies in different days, on the basis of the consistency definition data as illustrated in Fig. 11. For example, the contradiction information extracting unit 102 may extract, as contradiction information, information on whether or not there is variation in different days or a level of the variation, in the relationship between the chief complaint item data relating to the chief complaint of the consulter and the predetermined chief complaint evaluation item data. The contradiction information extracting unit 102 may identify a part in which the relationship between the chief complaint item data and the predetermined chief complaint evaluation item data varies in different days, and extract, as the contradiction information, the sleep-related data including the part for a plurality of days, based on the consistency definition data, for example.

The consistency definition data is data in which the consistency of data of items included in the sleep-related data (i.e. what is the basis for considering the items to be consistent) is defined. In this example, consistency definition data defining the consistency is prepared in advance, at least for the chief complaint item and the chief complaint evaluation item included in the sleep-related data. Fig. 11 represents, for example, as consistency definition data for "sleep onset latency" that is the item of the item ID = "f-1", a consistency determination criterion that is "±15 minutes" is registered. Fig. 11 also represents, for example, as consistency definition data for "daytime activity level" that is the item of the item ID = "j-1", a consistency determination criterion that is "±1" is registered. Although not illustrated in Fig. 1, the sleep improvement support device 100 according to the present exemplary embodiment may include a consistency definition data storage unit storing such consistency definition data.

The contradiction information extracting unit 102 may determine whether the chief complaint item data is consistent and whether the chief complaint evaluation item data is consistent between dates of the date pairs of the sleep-related data stored in the sleep-related data storage unit 104 on the basis of the consistency definition data illustrated in Fig. 11, for example, and extract contradiction information based on that result. For example, assume a case in which the sleep-related data as illustrated in Fig. 12 is stored in the sleep-related data storage unit 104.

First, the contradiction information extracting unit 102 identifies 10 date pairs as illustrated in (a) of Fig. 13, as the date pairs of the sleep-related data stored in the sleep-related data storage unit 104.

Next, the contradiction information extracting unit 102, for each of the identified date pair, determines whether the chief complaint item data (the sleep onset latency data in this example) is consistent and whether the chief complaint evaluation item data (the daytime activity level data in this example) is consistent between the relevant dates. For example, for the date pair of "12/1" and "12/2", the contradiction information extracting unit 102 determines that the sleep onset latency data is not consistent ("inconsistency") between the relevant dates, based on the consistency definition data for the sleep onset latency, on the grounds that the sleep onset latency indicated by the sleep onset latency data associated with the date information "12/1" is 90 minutes, the sleep onset latency indicated by the sleep onset latency data associated with the date information "12/2" is 20 minutes, and therefore the difference therebetween is -70 minutes. In addition, the contradiction information extracting unit 102 determines that daytime activity level data is consistent ("consistency") between the relevant dates, based on the consistency definition data for the daytime activity level, on the grounds that the daytime activity level indicated by the daytime activity level data associated with the date information "12/1" is seven, the daytime activity level indicated by the daytime activity level data associated with the date information "12/2" is seven, and therefore the difference therebetween is zero. The contradiction information extracting unit 102 performs similar processing to the remaining date pairs, thereby obtaining the determination result illustrated in (a) in Fig. 13.

Next, the contradiction information extracting unit 102, based on the obtained determination result, identifies the date pair in which the consistency of the chief complaint item data and the consistency of the chief complaint evaluation item data are different (refer to (b) of Fig. 13). Specifically, the contradiction information extracting unit 102 may extract, from the determination result illustrated in (a) of Fig. 13, the date pair of which the chief complaint item data is not consistent and the chief complaint evaluation item data is consistent, or the date pair of which the chief complaint item data is consistent and the chief complaint evaluation item data is not consistent. In this example, as illustrated in (b) of Fig. 13, the "12/1" and "12/2" date pair, "12/1" and "12/3" date pair, "12/1" and "12/5" date pair, "12/2" and "12/4" date pair, "12/3" and "12/4" date pair, as well as "12/4" and "12/5" date pair (total of six date pairs) are identified.

Lastly, the contradiction information extracting unit 102 extracts, as contradiction information, chief complaint item data (the sleep onset latency data in this example) and the chief complaint evaluation item data (the daytime activity level data in this example) in the identified date pairs.

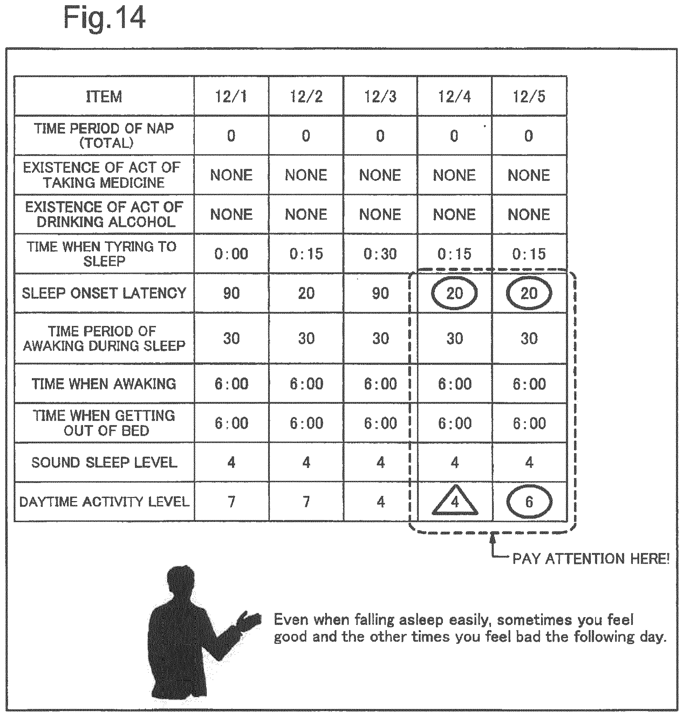

When contradiction information is extracted, while displaying, in emphasis, the extracted contradiction information in the listing table of sleep-related data used for extraction as illustrated in Fig. 14, the output unit 103 may output a non-correlation notification message 12, such as "Even when falling asleep easily, sometimes you feel good and the other times you feel bad the following day", notifying that even days on which the sleep states concerning the chief complaint exhibits a similar value may include days of which the chief complaint evaluation item data is good and days of which the chief complaint evaluation item data is bad. Fig. 14 illustrates an example of display in which favorability of each piece of data is exhibited, e.g., a piece of data included in the contradiction information which exhibits a favorable state is indicated by a circle mark, and a piece of data which does not is indicated by a triangle mark. Accordingly, how is the variation in the relationship between the chief complaint item data and the chief complaint evaluation item data (how they vary) and how much is the variation (how much they vary) can also be reported. In this example, too, the output unit 103 may only output the contradiction information in which the relevant parts are displayed in emphasis, without outputting any message. If the user is a doctor and the like, as long as the relevant parts are indicated, he or she can give an appropriate advice to the patient based thereupon. Fig. 14 illustrates an example in which the non-correlation notification message 12 is output by making use of the information on the latest date pair out of the extracted contradiction information. However, the number of date pair used for outputting the non-correlation notification message 12 is not limited to one. For example, all the extracted date pairs may be used, and selection may be performed one by one from the every combinations of the consistency of the chief complaint item data and the consistency of the chief complaint evaluation item data {consistent, inconsistent} {inconsistent, consistent}.

When the chief complaint item data is consistent, for example, the output unit 103 may output, as the non-correlation notification message 12, a message suggesting a possibility that the chief complaint may not be an important cause for the daytime activity, because the daytime state of the following day is not constant even when the sleep state corresponding to the chief complaint is at the similar level. When the chief complaint evaluation item data is consistent, for example, the output unit 103 may output, as the non-correlation notification message 12, a message suggesting a possibility that the chief complaint may not be an important cause for the daytime activity, because the daytime state of the following day is the similar level in either of cases in which the chief complaint arises and does not arise.

In the consistency definition, not only the difference in values but also information indicating that one of the pair is "admission" but the other of the pair is "non-admission" may also be considered. For example, in a case where it is deemed to be inconsistent if there is simply 30 minutes or more difference in the sleep onset latency, each day in a date pair will be determined not to have consistency if the data for the respective days is "15 minutes" and "60 minutes". However, even in a case in which the data for the relevant days is "60 minutes" and "90 minutes", the date pair is determined not to have consistency. Nevertheless, in the latter case, each day in the date pair is equivalent in a sense that both of the days undergo "difficulty falling asleep". In light of this, the definition may incorporate such propriety definition data as determining the date pair to be inconsistent, if one of the days is "admission" and the other of the days is "non-admission" and the difference therebetween is 30 minutes or more. It is also possible to use such a complex rule as stipulating that, with reference to a single piece of the consistency definition data, "if the data for the one of the days is within 30 minutes, the other is equal to or more than 30 minutes, and the difference therebetween is 30 minutes or more, the date pair is deemed to be inconsistent".

As stated in the above, according to the present exemplary embodiment, by comparing the sleep-related data of the plurality of days, and examining the consistency level of the relationship between the chief complaint item data and the chief complaint evaluation item data between the days (whether they are different between days), the non-correlation between the chief complaint and the quality of sleep is detected. Therefore, objective data is not necessarily needed. No sensor units, tests, and the like do not have to be used to obtain such objective data, and yet can easily make the consulter aware of his or her wrong cognition about the quality of his or her own sleep if the consulter has such wrong cognition.

In addition, when the relationship between the chief complaint item data and the chief complaint evaluation item data is not consistent between the days, by indicating, together with specific data, that even if there is a chief complaint related to insomnia, the chief complaint evaluation item data that would be susceptible in case of the sleep state corresponding to the chief complaint shows a favorable value, or that even days on which the chief complaint evaluation item data exhibits similar values may include a day on which sleep state concerning the chief complaint is good and a day on which the sleep state is bad. According to this, the effect of making a consulter aware of his or her way of thinking that is detrimental to insomnia can be further expected. The consulter, as his or her mental tendency, tends to overreact to the chief complaint and increase worries, which can be prevented by the present exemplary embodiment.

In the above-stated exemplary embodiment, "daytime activity level" is used as a chief complaint evaluation item; however the chief complaint evaluation item is not limited to "daytime activity level", and may be any other items as long as a consulter considers them worsening due to the insomnia. Example of the other chief complaint evaluation items include "fresh feelings in the morning", "how good is the makeup", "the conditions of the shadows under the eyes", "swelling" and " irritation level".

### Second Exemplary Embodiment

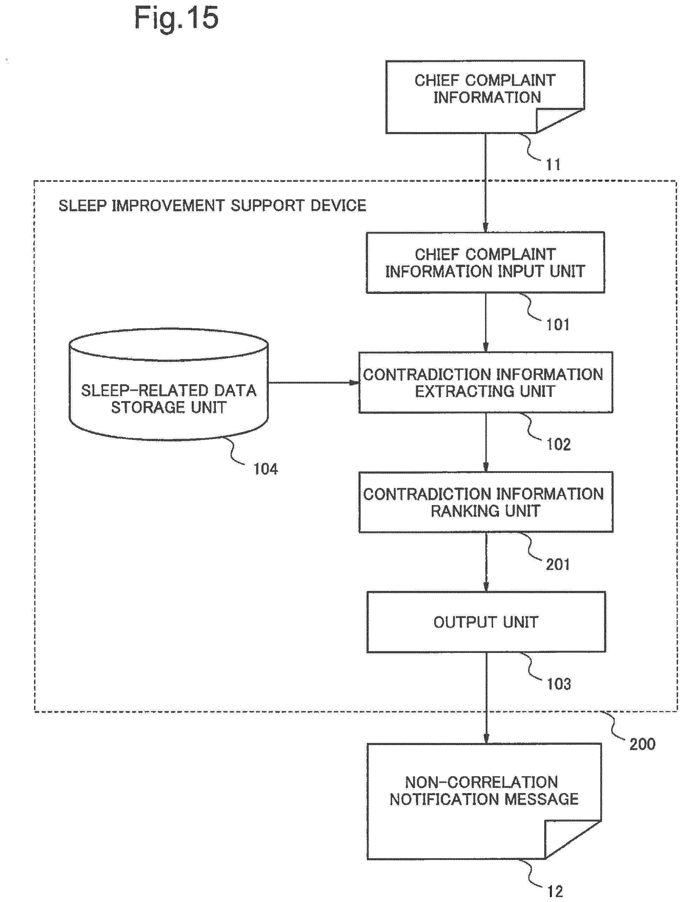

Fig. 15 is a block diagram illustrating an exemplary configuration of a sleep improvement support device according to the second exemplary embodiment of the present invention. The sleep improvement support device 200 illustrated in Fig. 15 is different from the sleep improvement support device 100 according to the first exemplary embodiment illustrated in Fig. 1, in that the sleep improvement support device 200 includes a contradiction information ranking unit 201.

The contradiction information ranking unit 201 assigns priority level to the extracted contradiction information, based on the magnitude of contradiction of that contradiction information, and ranks the pieces of contradiction information.

The contradiction information ranking unit 201 may assign the priority level based on the magnitude of contradiction of the extracted contradiction information, to that contradiction information, by deeming that there is a large magnitude of contradiction if there are more matching items other than the chief complaint item and the chief complaint evaluation item between the pieces of sleep-related data on dates in a combination of dates of the contradiction information, for example.

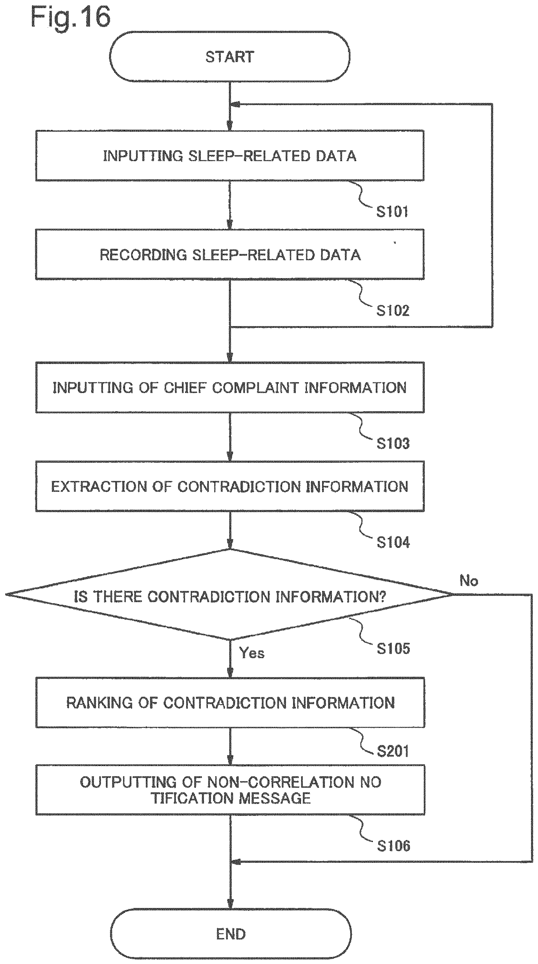

Next, the operation of the present exemplary embodiment is explained. Fig. 16 is a flowchart illustrating an exemplary operation of the present exemplary embodiment. Note that the same steps as in the first exemplary embodiment are assigned the same step numbers, and the explanation thereof is not made.

In the present exemplary embodiment, when the contradiction information extracting unit 102 extracts contradiction information (Yes in Step S105), the contradiction information ranking unit 201 assigns the priority level based on the magnitude of the contradiction of that contradiction information, and ranks the contradiction information (Step S201).

Next, the output unit 103 presents (outputs), to a user, contradiction information having a rank equal to or higher than the predetermined rank or having a priority level equal to or higher than a predetermined level based on the priority level assigned by the contradiction information ranking unit 201, and/or a non-correlation notification message 12 based on that contradiction information (Step S106).

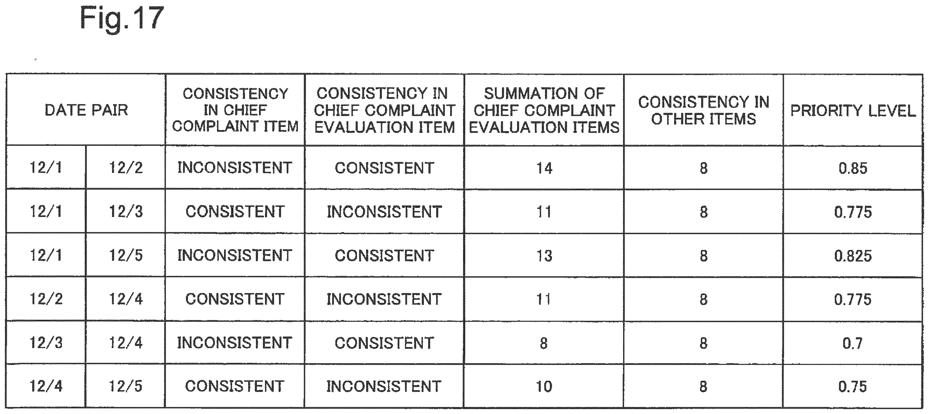

Fig. 17 is an explanatory diagram illustrating an example of priority level assigned by a contradiction information ranking means 201. Here, assume that, from among the sleep-related data illustrated in Fig. 12, the chief complaint item data and the chief complaint evaluation item data in the total of six date pairs as illustrated in Fig. 17 are extracted as contradiction information.

The contradiction information ranking unit 201 may compare, for each piece of extracted contradiction information, corresponding items, excluding the chief complaint items and the chief complaint evaluation items in dates of each date pair of the corresponding contradiction information, and assign a higher priority level to the date pair of contradiction information having more matching items, on the ground that the contradiction of the contradiction information is greater for date pairs having more matching items.

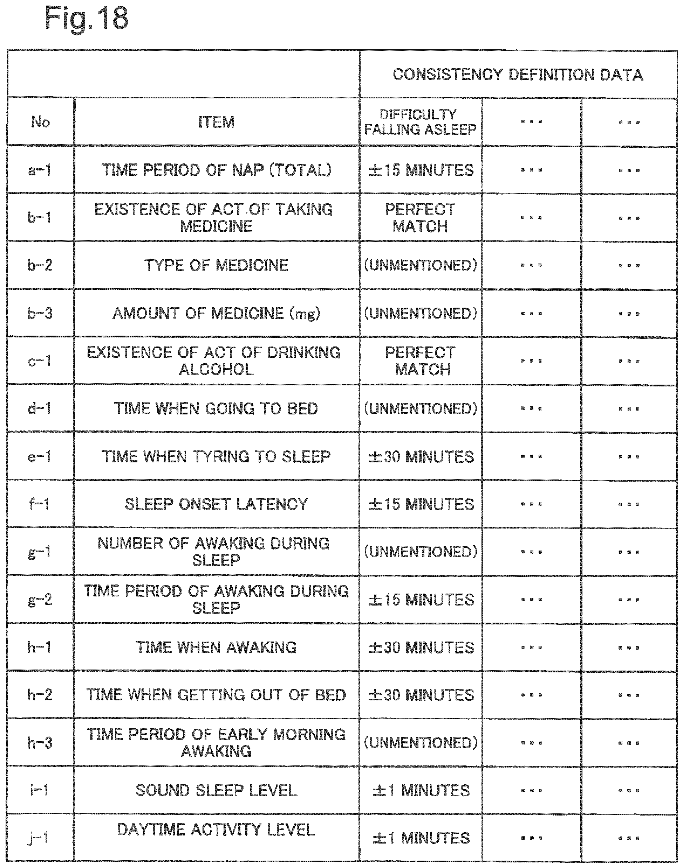

For example, based on the consistency definition data as illustrated in Fig. 18, the contradiction information ranking unit 201 may count, for each date pair of contradiction information, the number of items, excluding the chief complaint items and the chief complaint evaluation items, whose data matches (the number of matching items), and assign a higher priority level for the date pair having more matching items.

Fig. 18 is an explanatory diagram illustrating an example of consistency definition data according to the present example. Fig. 18 illustrates an example of consistency definition data defining, for each item included in the sleep-related data, the consistency of the data of the corresponding item. Fig. 18 illustrates an example of retaining consistency definition data separately for each chief complaint type. However, the consistency definition data may be common to all the chief complaint types. The "(UNMENTIONED)" in Fig. 18 indicates that consistency of the corresponding item is not considered.

The contradiction information ranking unit 201, for example, may count, for each date pair of contradiction information, the number of items, excluding the chief complaint items and the chief complaint evaluation items, whose data matches (the number of matching items), based on the consistency definition data as illustrated in Fig. 18, and, in addition, may derive the summation of the daytime activity levels, and assign a higher priority level for the date pair having a higher summation of daytime activity levels and more matching items.

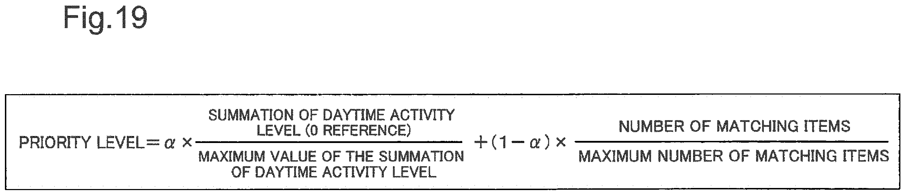

The contradiction information ranking unit 201 may derive, for example, the priority level of each piece of contradiction information by using the calculation expression illustrated in Fig. 19. Fig. 17 illustrates the calculation result of the priority levels when α=0.5 using the calculation expression illustrated in Fig. 19.

After each piece of contradiction information is assigned a priority level in such a manner, the contradiction information ranking unit 201, for example, may perform selection processing of , such as, selecting only the contradiction information being within a certain rank from the highest priority level or having a priority level equal to or higher than a predetermined threshold value ,(which includes selecting only the date pairs having a predetermined number or more of matching items). The contradiction information ranking unit 201 may, for example, have a devised configuration so as to select one having a higher priority level from among every combinations of the consistency of the chief complaint item data and the consistency of the chief complaint evaluation item data that are {consistent, inconsistent} or {inconsistent, consistent}, respectively. By doing so, the date pairs more similar in items other than the chief complaint items and the chief complaint evaluation items can be selected. It is also possible that the output unit 103 performs the above-described selection processing based on each of the priority levels assigned to pieces of contradiction information.

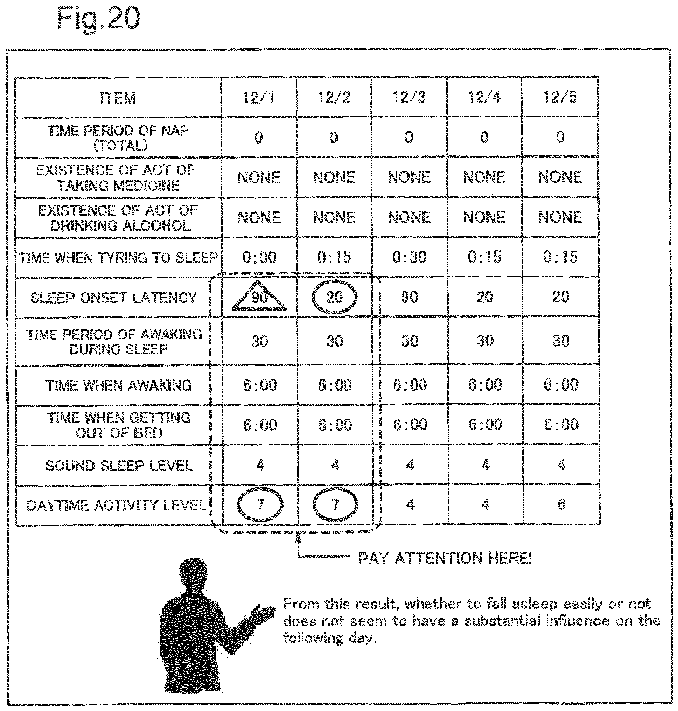

The output unit 103 displays, in emphasis, as illustrated in Fig. 20, for example, the contradiction information having the highest priority level, out of the contradiction information extracted in the listing table of sleep-related data used for extraction. The output unit 103 may output a non-correlation notification message 12, such as "From this result, whether to fall asleep easily or not does not seem to have a substantial influence on the following day.", reporting that even days on which values of the chief complaint evaluation item data are similar may include a day on which the sleep state concerning the chief complaint is good and a day the sleep state is bad. Fig. 20 also illustrates a piece of data included in the contradiction information which exhibit a favorable state is indicated by a circle mark, and those which do not is indicated by triangle mark, so that whether each piece of data is good or bad can be seen.

As described above, according to the present exemplary embodiment, by identifying data having a greater magnitude of contradiction, a non-correlation notification message 12 can be output to a user, to make the user more easily aware of the fact that the daytime activity is not related to the chief complaint, or that the chief complaint is not a major cause of the daytime activity.

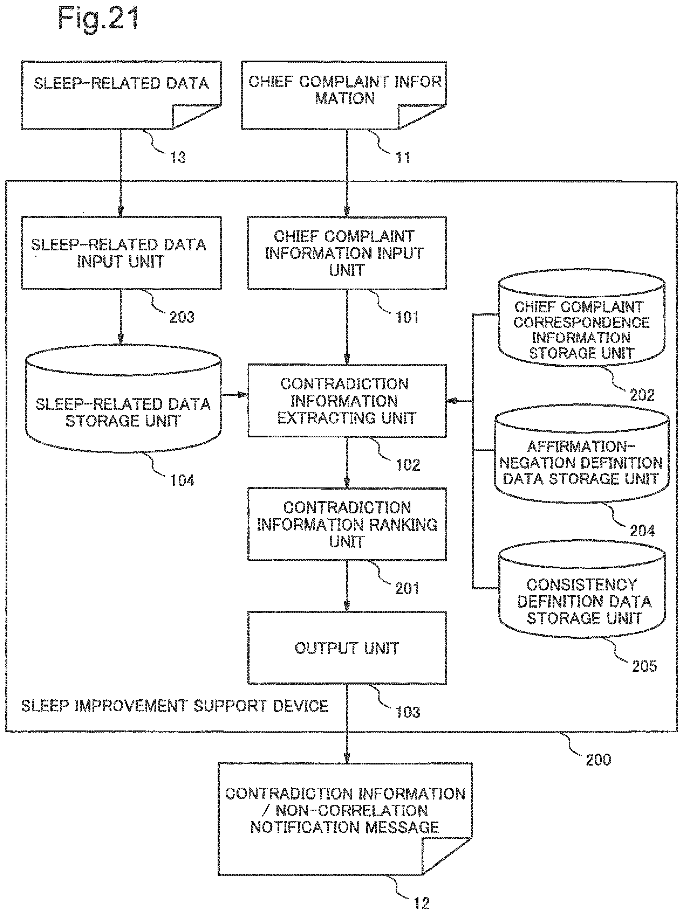

Fig. 21 is a block diagram illustrating another exemplary configuration of the sleep improvement support device according to the present exemplary embodiment. As illustrated in Fig. 21, the sleep improvement support device 200 according to the present exemplary embodiment may further include a chief complaint correspondence information storage unit 202 storing therein chief complaint correspondence information associating at least the chief complaint item being the relevant item in the sleep evaluation data included in the sleep-related data, with each of the chief complaint types determined in advance, similarly to the case of the first exemplary embodiment.

In addition, the sleep improvement support device 200 according to the present exemplary embodiment may further include a sleep-related data input unit 203 to let a user input sleep-related data, similarly to the case of the first exemplary embodiment.

The sleep improvement support device 200 according to the present exemplary embodiment may further include a propriety definition data storage unit 204 to store therein propriety definition data defining a affirmation-negation determining method for at least the chief complaint item and the chief complaint evaluation item included in the sleep-related data, similarly to the case of the first exemplary embodiment.

The sleep improvement support device 200 according to the present exemplary embodiment may further include a consistency definition data storage unit 205 to store therein consistency definition data defining the consistency of data of at least the chief complaint item and the chief complaint evaluation item included in sleep-related data, similarly to the case of the first exemplary embodiment.

The user of the present invention may be a consulter himself or herself, or a doctor or the like making a conversation with the consulter. If the user is a consulter, the CBT-I tool functions as a self-checking tool. Meanwhile, if the user is other than a consulter, the CBT-I tool functions as a tool that assists the doctor or the like making a conversation with the consulter. When operated by a person other than the consulter, the operator (user) may input information obtained from the consulter into the CBT-I tool, or give an advice or disclose data so as to remove anxiety of the consulter, with reference to messages displayed on the screen of the CBT-I tool.

### Industrial Applicability

Not limited to cases with insomnia, the present invention is suitably applicable to cases in which it is desired to easily detect the non-correlation relationship between a first phenomenon and a second phenomenon that would be susceptible to the occurrence of the first phenomenon.

As above, the present invention is described by using the above-described exemplary embodiments as examples. However, the present invention is not limited to the above-described exemplary embodiments. In other words, the present invention may be applied to various modes understood by those skilled in the art within the scope of the present invention.

The present application claims the priority based on Japanese Patent Application No. 2414-029709 filed on February 19, 2014, the entire disclosure of which is incorporated herein by reference.

### Reference Signs List

- 100, 200:: sleep improvement support device
- 101:: chief complaint information input unit
- 102:: contradiction information extracting unit
- 103:: output unit
- 104:: sleep-related data storage unit
- 201:: contradiction information ranking unit
- 202:: chief complaint correspondence information storage unit
- 203:: sleep-related data input unit
- 204:: affirmation-negation definition data storage unit
- 205:: consistency definition data storage unit

## Claims

1. A sleep improvement support device comprising:
sleep-related data storage means for storing, for two or more days, with date information, sleep-related data including sleep evaluation data for measuring a quality of sleep of a day and quality evaluation data that is influenced by the quality of sleep of the day, the sleep evaluation data being data of one or more items each of which indicates a state, a behavior, or a sense of a consulter, the quality evaluation data being data of one or more items each of which indicates a state, a behavior, or a sense of the consulter after rising;
chief complaint information input means for receiving, as input, chief complaint information indicating chief complaint of the consulter;
contradiction information extracting means for extracting contradiction information indicating variation, between days, in relationship between chief complaint item data and chief complaint evaluation item data, from the sleep-related data stored in the sleep-related data storage means, the chief complaint item data being an item of sleep evaluation data relating to the chief complaint of the consulter, the chief complaint evaluation item data being an item of quality evaluation data relating to the chief complaint of the consulter; and
output means for outputting the contradiction information extracted by the contradiction information extracting means and/or a message based on the contradiction information.

2. The sleep improvement support device according to Claim 1, wherein
the output means outputs a message indicating that there is no correlation between data of the main complaint item and data of the evaluation item, in addition to the extracted contradiction information and information indicating how relationship between the chief complaint item data and the chief complaint evaluation item data varies, the relationship being indicated by the extracted contradiction information.

3. The sleep improvement support device according to Claim 1 or Claim 2, comprising:
chief complaint correspondence information storage means for storing chief complaint correspondence information in which each of the chief complaint types determined in advance is at least associated with a chief complaint item, the chief complaint item being a relevant item in the sleep evaluation data included in the sleep-related data, wherein
the chief complaint information input means receives, as input, the chief complaint information including a chief complaint type of the chief complaint of the consulter.

4. The sleep improvement support device according to any one of Claim 1 to Claim 3, wherein
the contradiction information extracting means identifies a combination of dates on which relationship between the chief complaint item data and the chief complaint evaluation item data is different, in the sleep-related data stored in the sleep-related data storage means, and extracts, as contradiction information, the chief complaint item data and the chief complaint evaluation item data of the identified combination of dates.

5. The sleep improvement support device according to Claim 4, wherein
the contradiction information extracting means identifies, in the sleep-related data stored in the sleep-related data storage means, a combination of dates on which a similarity level of the chief complaint evaluation item data is equal to or more than a predetermined threshold value and a difference in the chief complaint item data is equal to or more than a predetermined threshold value, and extracts, as contradiction information, the chief complaint item data and the chief complaint evaluation item data of the identified combination of dates.

6. The sleep improvement support device according to Claim 4, comprising:
consistency definition data storage means for storing consistency definition data defining consistency of data of at least the chief complaint item and the chief complaint evaluation item included in the sleep-related data, wherein
the contradiction information extracting means identifies, based on the consistency definition data, from among combinations of dates of the sleep-related data stored in the sleep-related data storage means, a combination of dates on which consistency of the chief complaint item data and consistency of the chief complaint evaluation item data are different, and extracts, as contradiction information, the chief complaint item data and of the chief complaint evaluation item data in the identified combination of dates.

7. The sleep improvement support device according to any one of Claim 4 to Claim 6, comprising:
contradiction information ranking means for assigning, for each piece of the contradiction information which is extracted, a priority level based on a magnitude of contradiction of a piece of contradiction information, and ranking the piece of the contradiction information, wherein
the output means outputs a piece of the contradiction information having a priority level that is equal to or higher than a predetermined ranking or equal to or more than a predetermined level and/or a message based on the piece of the contradiction information, based on the priority level of each of the pieces of contradiction information assigned by the contradiction information ranking means.

8. The sleep improvement support device according to Claim 7, wherein
the contradiction information ranking means assigns, to the piece of the contradiction information which is extracted, the priority level based on the magnitude of the piece of the contradiction of contradiction information, by deeming that a contradiction is greater as a matching number of items other than the chief complaint item and the chief complaint evaluation item between pieces of sleep-related data on dates in a combination of dates of pieces of the contradiction information is greater.

9. The sleep improvement support device according to Claim 7, wherein
the chief complaint types includes at least "difficulty falling asleep", "awaking during sleep", "early morning awaking" or "trouble in sound sleep",
the quality evaluation data at least includes daytime activity level data indicating subjective evaluation of how much daytime activity can be done, as the chief complaint evaluation item data of each of the chief complaint types,
the contradiction information extracting means extracts the contradiction information indicating a variation, between days, in relationship between the chief complaint item data and the daytime activity level data, from the sleep-related data stored in the sleep-related data storage means, and
the contradiction information ranking means assigns the priority level based on the magnitude of the piece of the contradiction of extracted contradiction information to the piece of the contradiction information, by deeming that a contradiction is greater as the matching number of items other than the chief complaint item is greater and a summation of daytime activity level is higher in the combinations of dates of contradiction information.

10. The sleep improvement support device according to any one of Claim 1 to Claim 3, comprising:
affirmation-negation definition data storage means for storing affirmation-negation definition data defining an affirmation-negation determining method at least to a chief complaint item and a chief complaint evaluation item included in the sleep-related data, wherein
as a result of cross-tabulation of a day number of days of affirmation and negation of the chief complaint item data and a day number of days of affirmation and negation of the chief complaint evaluation item data, based on the affirmation-negation definition data, for a predetermined number of latest dates of sleep-related data stored in the sleep-related data storage means, if a day number of days on which affirmation and negation do not match between the chief complaint item data and the chief complaint evaluation item data is equal to or more than a predetermined threshold value, the contradiction information extracting means extracts the result of the cross-tabulation as the contradiction information.

11. A sleep improvement support method comprising:
in a state in which sleep-related data storage means stores, for two or more days, with date information, sleep-related data including sleep evaluation data for measuring a quality of sleep of a day and quality evaluation data that is influenced by the quality of sleep of the day, the sleep evaluation data being data of one or more items each of which indicates a state, a behavior, or a sense of a consulter, the quality evaluation data being data of one or more items each of which indicates a state, a behavior, or a sense of the consulter after rising,
when chief complaint information indicating chief complaint of the consulter is received as input,
extracting, by an information processing device, contradiction information indicating variation, between days, in relationship between chief complaint item data and chief complaint evaluation item data, from the sleep-related data stored in the sleep-related data storage means, the chief complaint item data being an item of sleep evaluation data relating to the chief complaint of the consulter, the chief complaint evaluation item data being an item of quality evaluation data relating to the chief complaint of the consulter; and
outputting, by the information processing device, the contradiction information extracted and/or a message based on the contradiction information.

12. A program storage medium storing a sleep improvement support program causing a computer capable of accessing sleep-related data storage means storing, for two or more days, with date information, sleep-related data including sleep evaluation data for measuring a quality of sleep of a day and quality evaluation data that is influenced by the quality of sleep of the day, the sleep evaluation data being data of one or more items each of which indicates a state, a behavior, or a sense of a consulter, the quality evaluation data being data of one or more items each of which indicates a state, a behavior, or a sense of the consulter after rising, to execute:
chief complaint information input processing of receiving, as input, chief complaint information indicating chief complaint of the consulter;
contradiction information extracting processing of extracting contradiction information indicating variation, between days, in relationship between chief complaint item data and chief complaint evaluation item data, from the sleep-related data stored in the sleep-related data storage means, the chief complaint item data being an item of sleep evaluation data relating to the chief complaint of the consulter, the chief complaint evaluation item data being an item of quality evaluation data relating to the chief complaint of the consulter; and
output processing of outputting the contradiction information extracted by the contradiction information extracting means and/or a message based on the contradiction information.
